# EUROPÄISCHE PATENTSCHRIFT

(11) **EP 0 720 608 B1**
(45) Veröffentlichungstag und Bekanntmachung des Hinweises auf die Patenterteilung: **09.07.1997**
(21) Anmeldenummer: 94928786.6
(22) Anmeldetag: 17.09.1994
(51) Int. Cl.: C07D 309/36, C07D 405/06, C07D 409/06, A61K 31/365

(54) **NEUE BISHISPIDINDERIVATE, VERFAHREN ZU IHRER HERSTELLUNG UND DIESE VERBINDUNGEN ENTHALTENDE ARZNEIMITTEL**
NOVEL DIHISPIDINE DERIVATIVES, PROCESS FOR PRODUCING THEM AND MEDICAMENTS CONTAINING THESE COMPOUNDS
NOUVEAUX DERIVES DE BISHISPIDINE, LEURS PROCEDES DE PREPARATION ET MEDICAMENTS CONTENANT CES COMPOSES

(30) Priorität: 22.09.1993 DE 4332203
(43) Veröffentlichungstag der Anmeldung: 10.07.1996
(73) Patentinhaber: BOEHRINGER MANNHEIM GMBH, 68298 Mannheim (DE)
(72) Erfinder: VOSS, Edgar, D-68519 Viernheim (DE); REITER, Rudolf, D-69469 Weinheim (DE); KILPERT, Claus, D-68305 Mannheim (DE)
(86) Internationale Anmeldenummer: EP9403116
(87) Internationale Veröffentlichungsnummer: WO9508547

(56) Entgegenhaltungen:
- CHEMICAL ABSTRACTS, vol. 116, no. 12, 1992, Columbus, Ohio, US; abstract no. 120411v, S.AMINOV ET AL. 'PHARMACOLOGY OF SOME FLAVONOIDS,LIGNANS AND CHALCONESRECOVERED FROM VARIOUS PLANTS OF CENTRAL ASIA.' Seite 20 ;

## Beschreibung

Gegenstand der vorliegenden Erfindung sind Methylenbishispidinderivate. Verfahren zu deren Herstellung und Arzneimittel, die diese Verbindungen enthalten.

Die Erfindung betrifft Methylenbishispidinderivate der allgemeinen Formel I 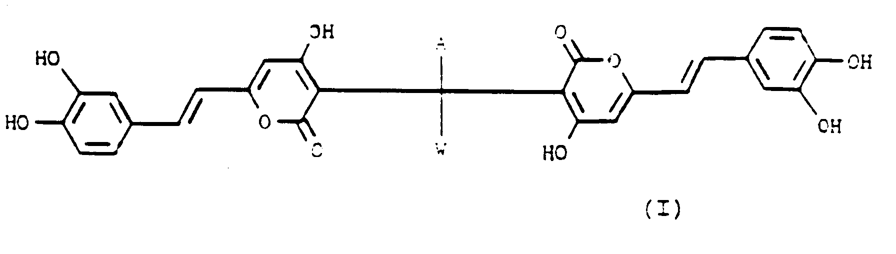 in welcher
- A: Wasserstoff, C₁-C₁₆-Alkyl, C₃-C₆-Cycloalkyl, eine Gruppe 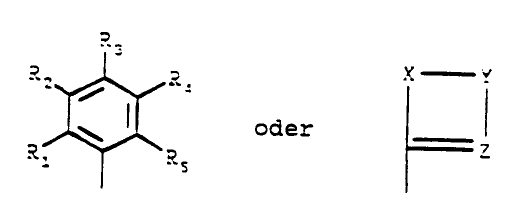 bedeutet, wobei
- R₁-R₅: gleichzeitig oder unabhängig voneinander Wasserstoff, C₁-C₆-Alkyl, Hydroxy, C₁-C₆-Acyloxy, C₁-C₆-Alkoxy, Benzyloxy, Halogen, Cyano, Carboxy, Amino, Phenyl, Alkylthio, Carboxyalkyl, Heteroalkyl oder eine Estergruppe sind.
- X: -CR₆=CR₇-, -CR₈=N-, -N=CR₉-, NR₁₀, Sauerstoff oder Schwefel ist,
- Y: -CR₆=CR₇-, -CR₁₁=N-, NR₁₀, Sauerstoff oder Schwefel ist,
- Z: =CR₁₁- oder Stickstoff ist,
wobei
- R₆-R₁₁: unabhängig voneinander Wasserstoff, Methyl, Halogen oder Carboxy bedeuten und
- W: Wasserstoff, C₁-C₄-Alkyl, gegebenenfalls durch Halogen substituiert. Arylalkyl, Carboxyl oder Carboxylester bedeuten
sowie deren physiologisch verträgliche Salze oder Ester.

Die Alkylreste in den genannten Alkyl-, Alkoxy-, Acyloxy-, Alkylthio-, Carboxyalkyl- oder Heteroalkylgruppen können geradkettig oder verzweigt sein. In der gegenwärtigen Erfindung bedeutet ein C₁-C₆-Alkylrest Methyl, Ethyl, Propyl, Isopropyl, Butyl, Isobutyl, t-Butyl, Pentyl oder Hexyl, Heteroalkyl bedeutet einen C₁-C₄-Alkylrest, substituiert durch Stickstoff, Sauerstoff oder Schwefel. Arylalkyl bedeutet eine gegebenenfalls durch Halogen, Hydroxy oder Methyl substituierte Phenylgruppe verknüpft durch einen C₁-C₄-Alkylrest. Carboxyalkyl ist eine Carboxygruppe verknüpft durch einen C₁-C₄-Alkylrest, Halogen bedeutet Fluor, Chlor, Brom oder Jod, vorzugsweise Chlor.

Überraschend wurde gefunden, daß die Verbindungen der Formel 1 wertvolle pharmakologische Eigenschaften aufweisen. Sie hemmen insbesondere die Bildung von AGE (Advanced Glycosylation Endproducts), deren Bedeutung für die Entstehung von diabetischen Spätkomplikationen gezeigt wurde (A. Cerami, Trends Biochem. Sci. **11**, 311 (1986)).

So kann die nichtenzymatische Glykosylierung von Plasmaproteinen durch deren Inkubation mit Glucose in vitro simuliert werden, die Reinjektion dieser Proteine führt in vivo zu typischen diabetischen Spätschäden (H. Vlassara et al., Diabetes **41**, Suppl. 1, 9A (1992)). AGE sind an der Verdickung der glomerulären Basismembran beteiligt, ein Prozeß verantwortlich für Niereninsuffizienz und Nierenversagen. Die nichtenzymatische Glykosylierung von Crystallin, einem Protein der Augenlinse. führt zu Änderungen der Tertiärstruktur und Polymerisation durch Oxidation von SH-Gruppen zu Disulfiden mit dem Resultat der diabetischen Kataraktbildung (V. Monnier, Clin. Endocrinol. Metab. **11**, 431 (1982)). Die durch Endprodukte der nichtenzymatischen Glykosylierung hervorgerufene Quervernetzung von Proteinen vermindert die Löslichkeit von Kollagen und ist an der Sklerose von Blutgefäßen beteiligt (H. Rosenburg et al.. Biochem. Biophys. Res. Commun. **91** 498 (1979)). Eine weitere Konsequenz ist das Einfangen von low density lipoproteins (M. Brownlee et al., Science 232, 1629 (1986)). Die Lokalisation dieser LDL-Proteine an das Endothelium stellt eine starke Stimulation atherosklerotischer Prozesse dar (D. Steinberg et al., J. Clin. Invest. **88**, 1785 (1991); D. Leake, Current Opinion in Lipidology, 2, 301 (1991)).

Verbindungen der Formel I sind daher geeignet zur Prophylaxe und Behandlung diabetischer Spätschäden (z. B. Retinopathie, Nephropathie und Neuropathie) sowie zur Prophylaxe und Therapie von Atherosklerose und Arteriosklerose.

Bevorzugte Verbindungen der allgemeinen Formel I sind Verbindungen, in denen A Wasserstoff, C₁-C₆-Alkyl, C₃-C₆-Cycloalkyl oder eine Gruppe 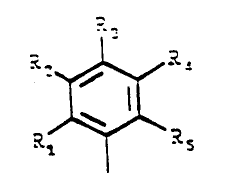 bedeutet, wobei R₁ und R₅ Wasserstoff sind.

Besonders bevorzugte Verbindungen der allgemeinen Formel I sind solche, bei denen R³ Wasserstoff, Hydroxy, C₁-C₄-Alkoxy, Cyano, Carboxy oder Halogen darstellt. R² und R⁴ unabhängig voneinander und unabhängig von R³ Wasserstoff.

Hydroxy, C₁-C₄-Alkyoxy oder C₁-C₄-Alkyl bedeuten, sowie R¹ und R⁵ Wasserstoffatome sind.

Besonders bevorzugte Verbindungen der allgemeinen Formel I sind auch solche, in denen A eine Gruppe 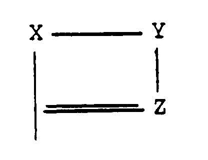 bedeutet, wobei
- X: -CH=CH-, NH, -CH=N, Sauerstoff oder Schwefel ist.
- Y: -CH=CH-, -CH=N- oder Schwefel ist und
- Z: =CH- oder Stickstoff bedeutet.

W ist in der allgemeinen Formel I bevorzugt Wasserstoff, C₁-C₄-Alkyl oder Carboxylester.

Die Verbindungen der allgemeinen Formel I, in der A und W die oben genannten Bedeutungen haben, werden hergestellt, indem Hispidin (II) mit einem Aldehyd oder Keton der Formel III kondensiert wird

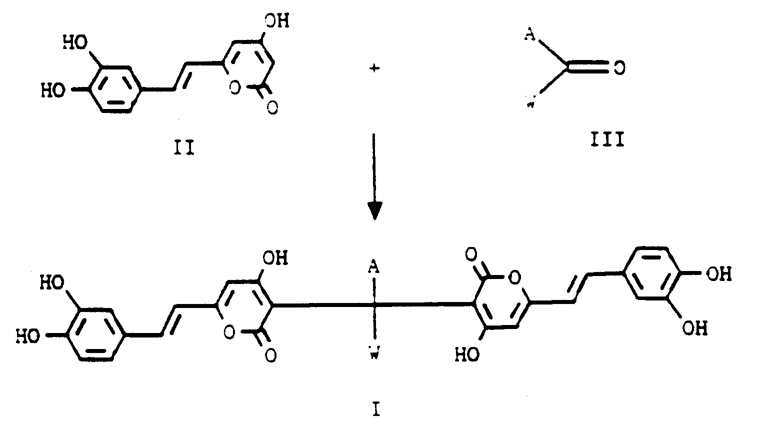

A und W besitzen dabei die oben genannten Bedeutungen. Die Reaktion kann durchgeführt werden durch Erhitzen der beiden Komponenten in einem polaren organischen Lösungsmittel, bevorzugt in protischen Lösungsmitteln wie einfachen aliphatischen Alkoholen, besonders Methanol oder Ethanol, in Gegenwart katalytischer Mengen einer Mineralsäure, beispielsweise Salzsäure oder Schwefelsäure. Die Umsetzung kann auch in einem dipolar aprotischen Lösungsmittel wie Dimethylformamid mit Zusatz von Piperidiniumacetat als Katalysator unter azeotroper Entwässerung mit Toluol durchgeführt werden.

Gewünschtenfalls können die hergestellten Verbindungen der allgemeinen Formel I, wenn sie sauer oder basischer Natur sind, in physiologisch verträgliche Salze überführt werden, und im Falle von Carbonsäuren ist ihre Umwandlung in Ester mit physiologisch unbedenklichen Alkoholen möglich. Zur Bildung von Salzen aus Carbonsäuren der allgemeinen Formel I eignen sich pharmakologisch verträgliche anorganische oder organische Basen wie z. B. Natriumhydroxid, Kaliumhydroxid, Calciumhydroxid, Methylglukamin, Morpholin oder Ethanolamin. Zur Salzbildung an Basen der allgemeinen Formel I eignen sich als Säuren z. B. Salzsäure, Schwefelsäure, Essigsäure, Citronensäure, Maleinsäure, Fumarsäure und Weinsäure.

Für den Fall, daß die Verbindungen der allgemeinen Formel I eine Carboxylfunktion enthalten, kommen als Ester dieser Carbonsäuren solche mit niederen einwertigen Alkoholen (wie z. B. Methanol oder Ethanol) oder mit mehrwertigen Alkoholen (wie z. B. Glycerin) in Frage.

Die in dem Verfahren zur Herstellung der Verbindungen der Formel I eingesetzte Ausgangsverbindung Hispidin (II) wurde nahezu gleichzeitig von R.L. Edwards et al. (Isolierung aus Polyporus hispidus: J. Chem. Soc. **1961**, 4995) und A. Ueno et al. (Isolierung aus Phaeolus schweinitzii; Chem. Pharm. Bull. **12**, 376 (1964)) entdeckt und die Struktur aufgeklärt.

Die durch Extraktion aus Basidiomyceten gewinnbaren Mengen an Hispidin sind aber viel zu gering, um als Ausgangsmaterial für weitergehende Synthesen und vertiefende pharmakologische Untersuchungen dienen zu können. Hieraus ergab sich die dringende Notwendigkeit zur Entwicklung eines leistungsfähigen chemischen Herstellungsverfahrens für Hispidin.

Eine erste aber wenig effiziente Hispidinsynthese wurde 1961 von Edwards et al. angegeben. Hierbei wird 3,4-Di(methoxymethoxy)benzaldehyd mit 4-Methoxymethoxy-6-methyl-2-pyron in Gegenwart von Magnesiummethanolat in Methanol umgesetzt, wobei die Ausbeute nur 2 % Hispidin beträgt, da ein außerordentlich komplexes Produktgemisch entsteht.

Als Alternative wird in der oben zitierten Publikation die Umsetzung von 3,4-Di(methoxymethoxy)benzaldehyd mit 4-Methoxy-6-methyl-2-pyron in Gegenwart von Magnesiummethanolat in Methanol angegeben. Die anschließende alkalische Verseifung und saure Recyclisierung führt zu einem derart komplexen Reaktionsgemisch, daß gebildetes Hispidin nur nach Acetylierung als Tri-O-Acetylverbindung isoliert werden kann (5 % Ausbeute). Die Gewinnung des freien Hispidins macht dann noch einen weiteren verlustreichen Schritt erforderlich.

Bei beiden von Edwards et al. angegebenen Verfahren erfordert die Darstellung der eingesetzten Ausgangsmaterialien außerdem den Einsatz des hochcarcinogenen Chlormethylethers, was eine industrielle Anwendung praktisch ausschließt.

Gegenstand der vorliegenden Erfindung ist nun ein Verfahren, welches auf der Umsetzung von 3,4-Dibenzyloxybenzaldehyd IV mit 4-Benzyloxy-6-methyl-2-pyron V in Gegenwart von Lithiumdiisopropylamid beruht. Die gebildete Aldolverbindung VI kann sehr schonend durch katalytische Hydrierung zur Tetrahydroxyverbindung VII debenzyliert werden. Die Dehydratisierung zu Hispidin II gelingt leicht mit verdünnter Säure. Das erfindungsgemäße Verfahren weist eine gut reproduzierbare Gesamtausbeute (IV -> II) von 30 % auf und ist damit den bisher bekannten Methoden zur Darstellung von Hispidin weit überlegen.

Zur Herstellung von Arzneimitteln werden die Verbindungen der allgemeinen Formel I in an sich bekannter Weise mit geeigneten pharmazeutischen Trägersubstanzen. Aroma-, Geschmacks- und Farbstoffen gemischt und beispielsweise als Tabletten oder Dragees ausgeformt oder unter Zugabe entsprechender Hilfsstoffe in Wasser oder Öl. wie z.B. Olivenöl. suspendiert oder gelöst.

Die Methylenbishispidinderivate der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z. B Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregelung oder Polyethylen-Derivate von Sorbitanhydriden.

Feste Trägerstoffe sind z. B. Stärke, Lactose, Mannit, Methylcellulose, Talkum, hochdisperse Kieselsäure, höhermolekulare Polymere (wie Polyethylenglykole). Für die orale Applikation geeignete Zubereitungen können gewünschtenfalls Geschmacks- und Süßstoffe enthalten.

Die verabreichte Dosis hängt vom Alter, der Gesundheit und dem Gewicht des Empfängers, dem Ausmaß der Krankehit, der Art gleichzeitiger gegebenenfalls durchgeführter weiterer Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage, die gegebenenfalls als Salze oder Ester vorliegen können:
1. 3,3'-(4-Hydroxybenzyliden)bis[6-[2-(3,4-dihydroxyphenyl)vinyl]-4-hydroxy-2H-pyran-2-on]
2. 3,3'-(2-Pyrrolylmethylen)bis[6-[2-(3,4-dihydroxyphenyl)vinyl]-4-hydroxy-2H-pyran-2-on]
3. 3,3'-(2-Phenylethyliden)bis[6-[2-(3,4-dihydroxyphenyl)vinyl]-4-hydroxy-2H-pyran-2-on]
4. 3,3'-(4-Carboxybenzyliden)bis[6-[2-(3,4-dihydroxyphenyl)vinyl]-4-hydroxy-2H-pyran-2-on]

### Ausführungsbeispiele

### Synthese von Hispidin (II):

### i) 4-Benzyloxy-6-methyl-2H-pyran-2-on (V)

Zur Lösung von 56.7 g (0.450 mol) 4-Hydroxy-6-methyl-2H-pyran-2-on und 1.0 g (0,004 mol) 18-Krone-6 in 900 ml wasserfreiem Dimethylformamid gab man nacheinander 124.4 g (0.900 mol) Kaliumcarbonat und 63 ml (0.547 mol) Benzylchlorid. Bei einer Innentemperatur von 70 - 75°C wurde 4.5 Stunden kräftig gerührt. Man saugte ab und wusch den Rückstand gut mit Essigester nach. Die vereinigten Filtrate wurden im Vakuum weitgehend eingedampft und anschließend auf Eiswasser gegossen. Der abgesaugte Niederschlag wurde nach dem Trocknen aus t-Butylmethylether umkristallisiert, 69.0 g (71 %) V. Schmp. 92-93°C.

### ii) 4-Benzyloxy-6-[2-(3,4-dibenzyloxyphenyl)-2-hydroxyethyl)]-2H-pyran-2-on (VI)

Unter Stickstoff wurde eine Lösung von 10.1 g (14.0 ml, 100 mmol) Diisopropylamin in 150 ml Tetrahydrofuran bei -78°C mit 41.0 ml (95 mmol) Butyllithium (2.3 M in n-Hexan) versetzt. Man ließ kurzfristig auf O°C kommen und kühlte sofort wieder auf -78°C ab. Nach Zutropfen einer Lösung von 19.0 g (88 mmol) 4-Benzyloxy-6-methyl-2H-pyran-2-on (V) in 100 ml wasserfreiem Tetrahydrofuran rührte man noch 45 Min. bei -78°C und tropfte anschließend eine Lösung von 12.8 g (40 mmol) 3,4-Dibenzyloxybenzaldehyd (IV) in 150 ml wasserfreiem Tetrahydrofuran zu. Man ließ die Innentemperatur allmählich auf -30°C ansteigen und versetzte mit einem Überschuß ges. Ammoniumchloridlösung. Nach Ansäuern mit 6n HCl wurde mit Essigester extrahiert, die vereinigten organischen Phasen getrocknet und eingedampft. Reinigung des Rückstandes durch flash-Chromatographie an Kieselgel (Laufmittel: Essigester/Heptan 1:2) ergab 14.4 g (68 %) VI, Schmp. 109-110°C (Ether).

### iii) 4-Hydroxy-6-[2-(3,4-dihydroxyphenyl)-2-hydroxyethyl]- 2H-pyran-2-on (VII)

35.6 g (66.6 mmol) 4-Benzyloxy-6-[2-(3,4-dibenzyloxyphenyl)-2-hydroxy-ethyl)]-2H-pyran-2-on (VI) wurden in 1200 ml Ethanol mit 10 g W2-Raney-Nickel bei Raumtemperatur 6 h unter einem Wasserstoffdruck von 44 mbar hydriert. Nach Aufnahme von 4.4 l Wasserstoff wurde vom Katalysator abgesaugt und mehrmals mit Tetrahydrofuran nachgewaschen. Die eingedampften Filtrate ergaben nach Verreiben mit Ether 12.2 g (70 %) VII Schmp. 153°C (Zers.).

### iv) 6-[2-(3,4-dihydroxyphenyl)vinyl]-4-hydroxy-2H-pyran-2-on (Hispidin), (II)

Zur Suspension aus 8.0 g (30 mmol) 4-Hydroxy-6-[2-(3,4-dihydroxyphenyl)-2- hydroxyethyl]-2H-pyran-2-on VII und 300 ml Tetrahydrofuran gab man 2 ml 3 N HCl. Die resultierende klare Lösung wurde 2 - 3 Stunden unter Rückfluß erhitzt, bis kein Ausgangsmaterial mehr detektiert werden konnte (DC-Kontrolle, Laufmittel: Toluol/Ethylformiat/Methanol 5:4:1, 0.5 % Ameisensäure). Nach Trocknung über Natriumsulfat wurde die Lösung stark eingedampft (Restvolumen ca. 50 ml) und durch Versetzen mit Ether zur Kristallisation gebracht. Ausbeute 4.7 g (63 %) Hispidin II, Schmp. 232 - 234°C (Zers.) bzw. Schmp. 253°C (Zers.: Nadelbüschel aus Toluol/Ethylformiat).

### Synthese der Methylenbishispidinderivate der allgemeinen Formel I:

### Beispiel 1:

### 3,3'-(4-Chlorbenzyliden)bis[6-[2-(3,4-dihydroxyphenyl)vinyl]-4-hydroxy-2H-pyran-2-on] (1)

Zur Lösung von 7.0 g (0.05 mol) 4-Chlorbenzaldehyd in 100 ml Methanol gab man 4.9 g (0.02 mol) Hispidin und versetzte anschließend mit 2.0 ml Salzsäure. Die Mischung wurde bei 50°C bis zum vollständigen Umsatz gerührt (DC-Verlaufskontrolle. Laufmittel: Toluol/Ameisensäureethylester/Ameisensäure 5:4:1). Man rührte die Reaktionsmischung in 500 ml Wasser ein und saugte den bräunlichen Niederschlag ab (Rohausbeute 6.1 g). Zur Reinigung wurde aus Essigester/Heptan umgefällt, 3.1 g (50%), Schmp. 193°C (Zers.).

### Beispiel 2:

### 3,3'-(4-Methylenbenzyliden)bis[6-[2-(3,4-dihydroxyphenyl)vinyl]-4-hydroxy-2H-pyran-2-on] (2)

Analog zu Beispiel 1 aus Hispidin und 4-Methylbenzaldehyd, nach Reinigung des abgesaugten Rohproduktes durch flash-Chromatographie an Kieselgel (Laufmittel: Toluol/Dioxan/Eisessig 72:20:8) wurde die erhaltene Verbindung noch mit Ether ausgerührt. Ausbeute 43 %, Schmp. 192°C (Zers.).

### Beispiel 3:

### 3,3'-(3-Hydroxy-4-methoxybenzyliden)bis[6-[2-(3,4-dihydroxyphenyl)vinyl]-4-hydroxy-2H-pyran-2-on] (3)

Analog zu Beispiel 2 aus Hispidin und 3-Hydroxy-4-methoxybenzaldehyd. Laufmittel: Toluol/Ethylformiat/Methanol 5:4:1, 0.5 % Ameisensäure. Ausbeute: 36 %, Schmp. 181°C (Zers.).

### Beispiel 4:

### 3,3'-[4-Hydroxy-3,5-(bis-1,1-dimethylethyl)benzyliden]bis [6-[2-(3,4-dihydroxyphenyl)vinyl]-4-hydroxy-2H-pyran-2-on] (4)

Analog zu Beispiel 2 aus Hispidin und 4-Hydroxy-3,5-(bis-1,1-di-methylethyl)benzaldehyd. Laufmittel: Toluol/Dioxan/Eisessig 72:20:8) Ausbeute: 40%, Schmp. 148°C (Zers.).

### Beispiel 5:

### 3,3'-(4-Methoxybenzyliden)bis[6-[2-(3,4-dihydroxyphenyl)vinyl]-4-hydroxy-2H-pyran-2-on] (5)

Analog zu Beispiel 2 aus Hispidin und 4-Methoxybenzaldehyd, Laufmittel: Toluol/Ethylformiat/Methanol 5:4:1, 1% Ameisensäure. Ausbeute 28%. Schmp. 167°C (Zers.).

### Beispiel 6:

### 3,3'-(3,4-Dimethoxybenzyliden)bis[6-[2-(3,4-dihydroxyphenyl)vinyl]-4-hydroxy-2H-pyran-2-on) (6)

Analog zu Beispiel 2 aus Hispidin und 3,4-Dimethoxybenzaldehyd. Laufmittel: Toluol/Ethylformiat/Methanol 5:4:1, 0.5% Ameisensäure, Ausbeute 32%, Schmp. 177°C (Zers.).

### Beispiel 7:

### 3,3'-(3,4-Dihydroxybenzyliden)bis[6-(2-(3,4-dihydroxyphenyl)vinyl)-4-hydroxy-2H-pyran-2-on] (7)

Analog zu Beispiel 2 aus Hispidin und 3,4-Dihydroxybenzaldehyd. Laufmittel: Toluol/Ethylformiat/Methanol 5:4:1, 1% Ameisensäure. Ausbeute: 41%, Schmp. 238°C (Zers.).

### Beispiel 8:

### 3,3'-(4-Cyanobenzyliden)bis[6-[2-(3,4-dihydroxyphenyl)vinyl]-4-hydroxy-2H-pyran-2-on] (8)

Analog zu Beispiel 1 aus Hispidin und 4-Cyanobenzaldehyd. Umkristallisiert aus Essigester/Heptan, Ausbeute 41%, Schmp. 198°C (Zers.).

### Beispiel 9:

### 3,3'-Benzylidenbis-[6-[2-(3,4-dihydroxyphenyl)vinyl]-4-hydroxy-2H-pyran-2-on] (9)

Analog zu Beispiel 2 aus Hispidin und Benzaldehyd. Laufmittel: Toluol/Ethylformiat/Methanol 5:4:1, 0.5% Ameisensäure. Ausbeute: 33%, Schmp. 163°C (Zers.).

### Beispiel 10:

### 3,3'-Methylenbis[6-[2-(3,4-dihydroxyphenyl)vinyl]-4-hydroxy-2H-pyran-2-on] (10)

Analog zu Beispiel 2 aus 1.00g (4.00 mmol) Hispidin und 15 ml (O.2 mol) 35%iger Formalinlösung. Umgefällt aus Tetrahydrofuran/Isohexan. Ausbeute 28%, Schmp. 290°C (Zers.)

### Beispiel 11:

### 3,3'-(2-Thienylmethylen)bis[6-[2-(3,4-dihydroxyphenyl)vinyl]-4-hydroxy-2H-pyran-2-on] (11)

Analog zu Beispiel 2 aus Hispidin und Thiophen-2-aldehyd. Laufmittel: Toluol/Ethylformiat/Methanol 5:4:1, 0.5% Ameisensäure. Ausbeute 31%, Schmp. >300°C.

### Beispiel 12:

### 3,3'-(2-Pyridylmethylen)bis[6-[2-(3,4-dihydroxyphenyl)vinyl]-4-hydroxy-2H-pyran-2-on]hydrochlorid (12)

Analog zu Beispiel 1 aus Hispidin und Pyridin-2-aldehyd. Der abgesaugte Niederschlag wurde intensiv mit Essigester gewaschen. Ausbeute 55%, Schmp. 228°C (Zers.).

### Beispiel 13:

### 3,3'-(3,4,5-Trimethoxybenzyliden)bis[6-[2-(3,4-dihydroxyphenyl)vinyl]-4-hydroxy-2H-pyran-2-on) (13)

Analog zu Beispiel 2 aus Hispidin und 3,4,5-Trimethoxybenzaldehyd. Laufmittel: Toluol/Ethylformiat/Methanol 5:4:1, 0.5% Ameisensäure, Ausbeute 30%, Schmp. 208°C (Zers.).

### Beispiel 14:

### 3,3'-(3-Thienylmethylen)bis[6-[2-(3,4-dihydroxyphenyl)vinyl]-4-hydroxy-2H-pyran-2-on] (14)

Analog zu Beispiel 2 aus Hispidin und Thiophen-3-aldehyd. Laufmittel: Toluol/Ethylformiat/Methanol 5:4:1, 0.5% Ameisensäure. Ausbeute 35%, Schmp. 243°C (Zers.).

### Beispiel 15:

### 3,3'-(1-Hexyliden)bis[6-[2-(3,4-dihydroxyphenyl)vinyl]-4-hydroxy-2H-pyran-2-on] (15)

Analog zu Beispiel 2 aus 1.00 g (4.00 mmol) Hispidin und 9.6 ml (80 mmol) Hexanal. Laufmittel: Toluol/Ethylformiat/Methanol 5:4:1, 0.5% Ameisensäure. Ausbeute 45%, Schmp. 109°C (Zers.).

### Beispiel 16:

### 3,3'-(2-Imidazolylmethylen)bis[6-[2-(3,4-dihydroxyphenyl)vinyl]-4-hydroxy-2H-pyran-2-on]hydrochlorid (16)

Analog zu Beispiel 2 aus Hispidin und Imidazol-2-aldehyd. Laufmittel: Toluol/Ethylformiat/Methanol 5:4:1, 0.5% Ameisensäure, Ausbeute 4%, Schmp. 147°C (Zers.).

### Beispiel 17:

### 3,3'-(4-Brombenzyliden)bis[6-[2-(3,4-dihydroxyphenyl)vinyl]-4-hydroxy-2H-pyran-2-on] (17)

Analog zu Beispiel 2 aus Hispidin und 4-Brombenzaldehyd. Laufmittel: Toluol/Ethylformiat/Methanol 5:4:1, 0.5% Ameisensäure, Ausbeute 27%, Schmp. 228°C (Zers.).

### Beispiel 18:

### 3,3'-(4-Benzyloxybenzyliden)bis[6-[2-(3,4-dihydroxyphenyl)vinyl]-4-hydroxy-2H-pyran-2-on] (18)

Analog zu Beispiel 2 aus Hispidin und 4-Benzyloxybenzaldehyd. Laufmittel: Toluol/Ethylformiat/Methanol 5:4:1, 0.5% Ameisensäure, Ausbeute 11%, Schmp. 180°C (Zers.).

### Beispiel 19:

### 3,3'-Methoxycarbonylmethylenbis[6-[2-(3,4-dihydroxyphenyl)vinyl]-4-hydroxy-2H-pyran-2-on] (19)

Analog zu Beispiel 2 aus Hispidin und Glyoxylsäure, Laufmittel: Toluol/Ethylformiat/Methanol 5:4:1, 0.5% Ameisensäure, Ausbeute 18%, Schmp. 203°C (Zers.).

### Beispiel 20:

### 3,3'-(Cyclohexylmethylen)bis[6-[2-(3,4-dihydroxyphenyl)vinyl]-4-hydroxy-2H-pyran-2-on] (20)

Analog zu Beispiel 2 aus 1.00 g (4.00 mmol) Hispidin und 6.7 g (60 mmol) Cyclohexanaldehyd. Reinigung des abgesaugten Niederschlages durch flash-Chromatographie an Kieselgel (Laufmittel: Toluol/Ethylformiat/Methanol 5:4:1, 0.5% Ameisensäure, Ausbeute: 36%, Schmp. 154°C (Zers.).

### Versuchsbeschreibung

### Prüfung auf AGE-Bildung inhibierende Aktivität

Lysozym wird in einer Endkonzentration von 20 mg/ml in 100 mmol/L Kaliumphosphatpuffer, pH 7.4 gelöst, 5 mmol/L Natriumazid wird als Konservierungsmittel zugesetzt. Die AGE-Bildung wird durch den Zusatz von 200 mmol/L Glukose oder Xylose initiiert. Prüfsubstanzen oder die Referenzsubstanz Aminoguanidin werden zur Quantifizierung einer die AGE-Bildung hemmenden Aktivität in unterschiedlichen Konzentrationen eingesetzt. Losungsmittelkontrollen sowie Kontrollansätze in der Gegenwart von 200 mmol/L Sorbit oder Xylit werden zu Korrekturzwecken mitgeführt. Die Inkubationsdauer der Reaktionsansätze liegt in Abhängigkeit vom eingesetzten Zucker zwischen 24 und 120 Stunden und ist so gewählt, daß überwiegend Dimere und nur in geringem Außmaß andere, höhermolekulare Oligomerisierungsprodukte gebildet werden.

Die Vorbereitung der Proben und die Durchführung der SDS-Gelelektrophorese erfolgt nach Literaturangaben. Kurz zusammengefaßt werden die Proben mit der 4fachen Menge an Probenpuffer (50 mmol/L Tris* Acetat, pH 7.5, 1 % SDS, 5 mmol/L Dithiotreit) verdünnt und für 3 Minuten auf 95 °C erhitzt. Aliquots werden auf 8 - 18 % Gradient-SDS-Gele aufgetragen. Zur Molekulargewichtsbestimmung werden Molekulargewichtsmarker im Bereich 14-340 kD mitgeführt. Die Auftrennung nach Molekulargewicht erfolgt unter folgenden Laufbedingungen: Spannung: 600 V, Stromstärke: 50 mA, Leistung: 30 W, Laufdauer: ∼ 60 Minuten.

Nach der Elektrophorese weden die Gele nach Standardverfahren fixiert und mit Comassie Blue gefärbt. Nach der Entfärbung über Nacht und Trocknung werden die Gele densitometrisch (Pharmacia UltraScan XL mit GelScan XL Software) vermessen.

Zur Ermittlung der Schwellenkonzentration und der IC₅₀-Werte einer AGE-inhibierenden Wirkung von Prüfsubstanzen wird die Dimerenbildung halblogarithmisch gegen die Prüfkonzentration der Substanz aufgetragen und die oben genannten Kenngrößen rechnerisch ermittelt.

**Tabelle**

| **Verbindung Bsp. Nr.** | **Schwellenkonzentration [µmol/L]** | **IC50 [µmol/L]** |
|---|---|---|
| 1 | > 1 | 4 |
| 3 | > 1 | 2 |
| 4 | > 1 | 2 |
| 5 | > 1 | 2 |
| 6 | > 1 | 5 |
| 7 | 2 | 4 |
| 8 | 10 | 22 |
| Aminoguanidin | 500 | 4 500 |

## Patentansprüche

1. Methylenbishispidinderivate der allgemeinen Formel I 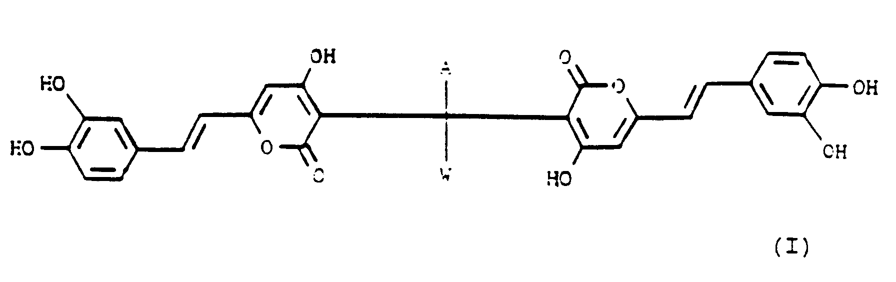 in der
A Wasserstoff, C₁-C₁₆-Alkyl, C₃-C₆-Cycloalkyl, eine Gruppe 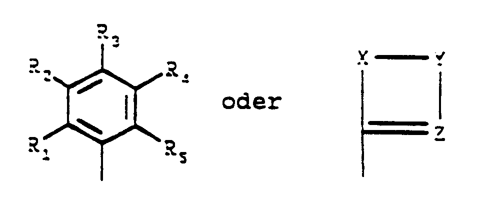 bedeutet, wobei
R₁-R₅ gleichzeitig oder unabhängig voneinander Wasserstoff, C₁-C₆-Alkyl, Hydroxy, C₁ -C₆-Acyloxy, C₁-C₆-Alkoxy, Benzyloxy, Halogen, Cyano, Carboxy, Amino, Phenyl, Alkylthio, Carboxyalkyl, Heteroalkyl oder eine Estergruppe sind.
X -CR₆=CR₇-, -CR₈=N-, -N=CR₉-, NR₁₀, Sauerstoff oder Schwefel ist.
Y -CR₆=CR₇-, -CR₁₁=N-, NR₁₀, Sauerstoff oder Schwefel ist.
Z CR₁₁ oder Stickstoff ist.
wobei
R₆-R₁₁ unabhängig voneinander Wasserstoff, Methyl, Halogen oder Carboxy bedeuten und
W Wasserstoff, C₁-C₄-Alkyl, gegebenenfalls durch Halogen substituiert. Arylalkyl, Carboxyl oder Carboxylester bedeuten.
sowie deren physiologisch verträgliche Salze oder Ester.

2. Methylenbishispidinderivate nach Anspruch 1, dadurch gekennzeichnet, daß
A Wasserstoff, C₁-C₆-Alkyl, C₃-C₆-Cycloalkyl, eine Gruppe 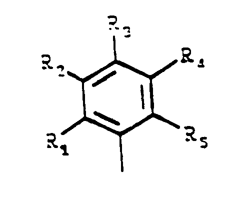 bedeutet, wobei R₁ und R₅ Wasserstoff sind.

3. Methylenbishispidinderivate nach Anspruch 2, dadurch gekennzeichnet, daß R₃ Wasserstoff, Hydroxy, C₁ -C₄-Alkoxy, Cyano, Carboxy oder Halogen bedeutet, R₂ und R₄ unabhängig voneinander und unabhängig von R₃ Wasserstoff, Hydroxy, C₁-C₄-Alkoxy oder C₁-C₄-Alkyl bedeuten.

4. Methylenbishispidinderivate nach Anspruch 1, dadurch gekennzeichnet, daß A eine Gruppe 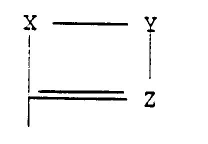 bedeutet, wobei
X -CH=CH-, NH, -CH=N-, Sauerstoff oder Schwefel ist.
Y -CH=CH-, -CH=N- oder Schwefel ist und
Z CH- oder Stickstoff bedeutet.

5. Methylenbishispidinderivate nach einem der Ansprüche 1 bis 4, dadurch gekennzeichnet, daß W Wasserstoff, C₁-C₄-Alkyl oder Carboxylester bedeutet.

6. Arzneimittel enthaltend mindestens ein Methylenbishispidinderivat nach einem der Ansprüche 1 bis 5 und übliche Träger- und Hilfsstoffe.

7. Verwendung von Methylenbishispidinderivaten nach einem der Ansprüche 1 bis 5 zur Herstellung von Arzneimitteln zur Prophylaxe und Therapie diabetischer Spätschäden, Atherosklerose und Arteriosklerose.

8. Verfahren zur Herstellung von Methylenbishispidinderivaten der allgemeinen Formel I nach einem der Ansprüche 1 bis 5, dadurch gekennzeichnet, daß Hispidin (Formel II) 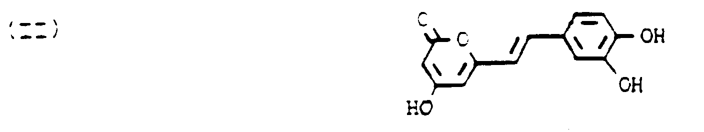 mit einem Aldehyd oder Keton der Formel III 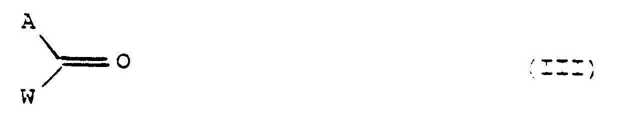 in der A und W die genannten Bedeutungen besitzen, umgesetzt wird durch Erhitzen in einem polaren organischen Lösungsmittel in Gegenwart katalytischer Mengen einer Mineralsäure oder in einem dipolar aprotischen Lösungsmittel in Gegenwart katalytischer Mengen von Piperidiniumacetat unter azeotroper Entwässerung.

9. Verfahren nach Anspruch 8, dadurch gekennzeichnet, daß Hispidin (II) hergestellt wird, indem man 3,4-Dibenzyloxybenzaldehyd (IV) 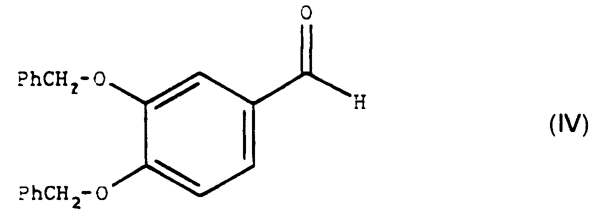 mit 4-Benzyloxy-6-methyl-2H-pyran-2-on (V) 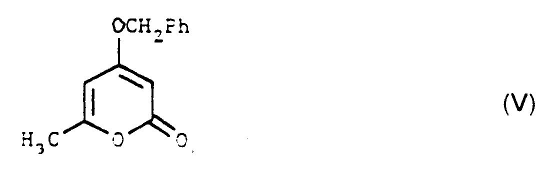 in Gegenwart von Lithiumdiisopropylamid zur Aldolverbindung VI umsetzt 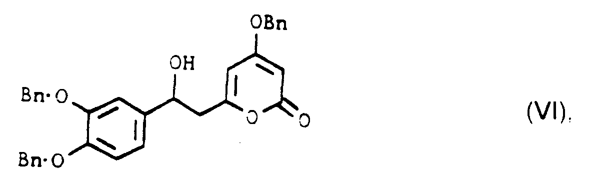 diese anschließend durch katalytische Hydrierung zur Tetrahydroxyverbindung VII debenzyliert 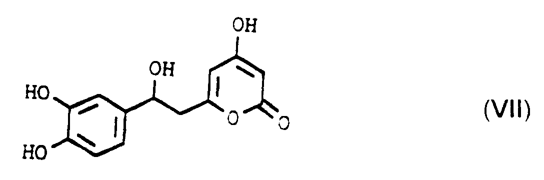 und zuletzt die Verbindung VII mit verdünnter Mineralsäure zu Hispidin (II) dehydratisiert.

## Claims

1. Methylene-bishispidine derivatives of the general formula I 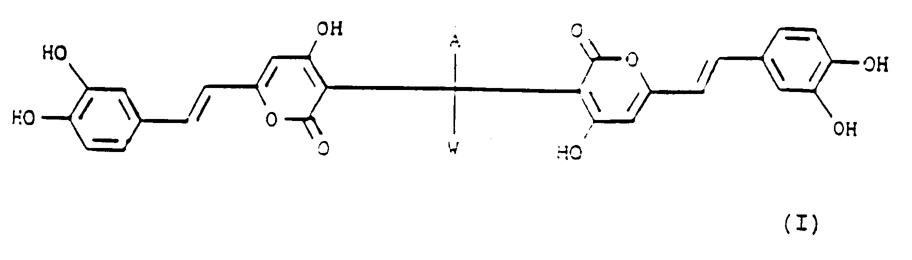 in which
A denotes hydrogen, C₁-C₁₆ alkyl, C₃-C₆ cycloalkyl, a group 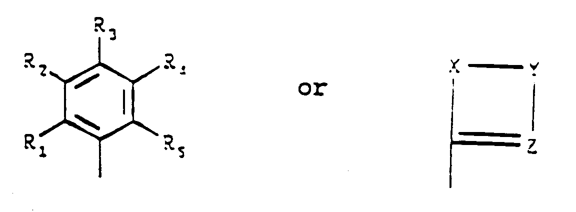 in which
R₁-R₅ simultaneously or independently of one another are hydrogen, C₁-C₆ alkyl, hydroxy, C₁-C₆ acyloxy, C₁-C₆ alkoxy, benzyloxy, halogen, cyano, carboxy, amino, phenyl, alkylthio, carboxyalkyl, heteroalkyl or an ester group,
X is -CR₆=CR₇-, -CR₈=N-, -N=CR₉-, NR₁₀, oxygen or sulphur,
Y is -CR₆=CR₇-, -CR₁₁=N-, NR₁₀, oxygen or sulphur
Z is CR₁₁ or nitrogen
in which
R₆-R₁₁ independently of one another denote hydrogen, methyl, halogen or carboxy and
W denotes hydrogen, C₁-C₄ alkyl, optionally substituted by halogen, arylalkyl, carboxyl or a carboxyl ester
as well as physiologically tolerated salts or esters thereof.

2. Methylene-bishispidine derivatives as claimed in claim 1, wherein
A denotes hydrogen, C₁-C₆ alkyl, C₃-C₆ cycloalkyl, a group 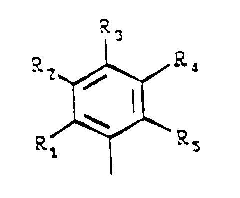 in which R₁ and R₅ are hydrogen.

3. Methylene-bishispidine derivatives as claimed in claim 2, wherein R₃ denotes hydrogen, hydroxy, C₁-C₄ alkoxy, cyano, carboxy or halogen, R₂ and R₄ independently of one another and independently of R₃ denote hydrogen, hydroxy, C₁-C₄ alkoxy or C₁-C₄ alkyl.

4. Methylene-bishispidine derivatives as claimed in claim 1, wherein A denotes a group 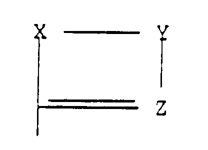 in which
X is -CH=CH-, NH, -CH=N-, oxygen or sulphur,
Y is -CH=CH-, -CH=N- or sulphur and
Z is CH- or nitrogen.

5. Methylene-bishispidine derivatives as claimed in one of the claims 1 to 4, wherein W denotes hydrogen, C₁-C₄ alkyl or a carboxyl ester.

6. Pharmaceutical agent containing at least one methylene-bishispidine derivative as claimed in one of the claims 1 to 5 and common carriers and auxiliary substances.

7. Use of methylene-bishispidine derivatives as claimed in one of the claims 1 to 5 for the production of pharmaceutical agents for the prophylaxis and therapy of late diabetic lesions, atherosclerosis and arteriosclerosis.

8. Process for the production of methylene-bishispidine derivatives of the general formula I as claimed in one of the claims 1 to 5, wherein hispidine (formula II) 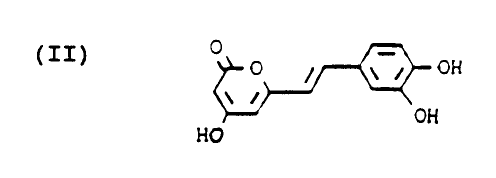 is reacted with an aldehyde or a ketone of formula III 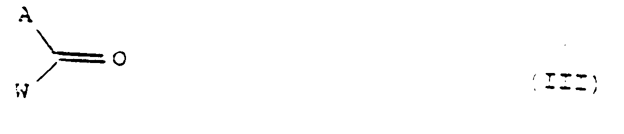 in which A and W have the stated meanings, by heating in a polar organic solvent in the presence of catalytic amounts of a mineral acid or in a dipolar aprotic solvent in the presence of catalytic amounts of piperidinium acetate under azeotropic dehydration.

9. Process as claimed in claim 8, wherein hispidine (II) is produced by reacting 3,4-dibenzyloxy-benzaldehyde (IV) 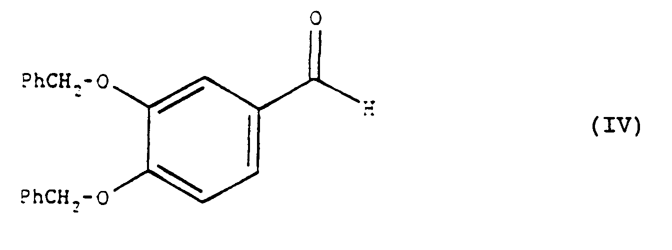 with 4-benzyloxy-6-methyl-2H-pyran-2-one (V) 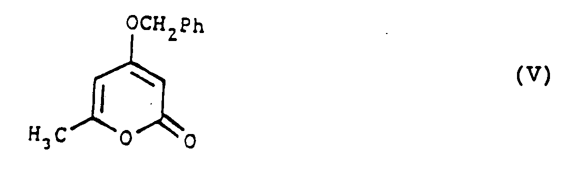 in the presence of lithium diisopropylamide to form an aldol compound VI 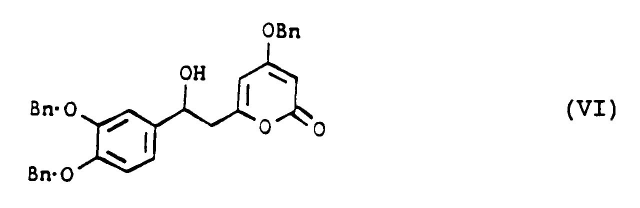 and subsequently debenzylating this by catalytic hydrogenation to form the tetrahydroxy compound VII 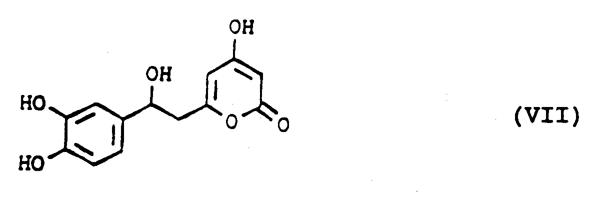 and finally dehydrating the compound VII with dilute mineral acid to form hispidine (II).

## Revendications

1. Dérivés de méthylènebishispidine de formule générale I 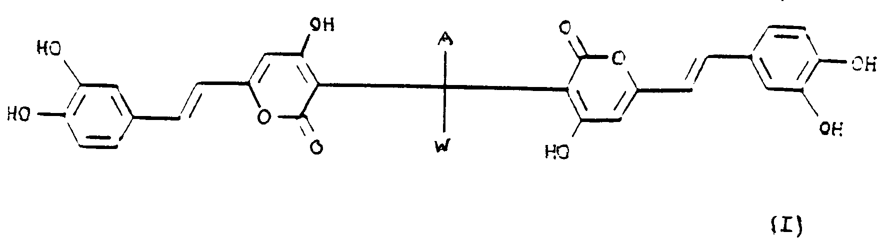 dans laquelle
A représente un atome d'hydrogène, un groupe alkyle en C₁-C₁₆, cycloalkyle en C₃-C₆, un groupe 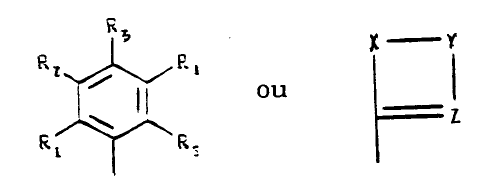 où
R₁ à R₅ représentent, simultanément ou indépendamment, un atome d'hydrogène, un groupe alkyle en C₁-C₆, hydroxy, acyloxy en C₁-C₆, alcoxy en C₁-C₆, benzyloxy, halogéno, cyano, carboxy, amino, phényle, alkylthio, carboxyalkyle, hétéroalkyle ou ester,
X représente un groupe -CR₆=CR₇-, -CR₈=N-, -N=CR₉-, NR₁₀, un atome d'oxygène ou de soufre,
Y représente un groupe -CR₆=CR₇-, -CR₁₁=N-, NR₁₀, un atome d'oxygène ou de soufre,
Z représente CR₁₁ ou un atome d'azote,
où
R₆ à R₁₁ représentent, indépendamment, un atome d'hydrogène, un groupe méthyle, halogéno ou carboxy, et
W représente un atome d'hydrogène, un groupe alkyle en C₁-C₄ éventuellement substitué par un halogène, un groupe aralkyle, carboxyle ou carboxylester,
ainsi que leurs sels ou esters physiologiquement acceptables.

2. Dérivés de méthylènebishispidine selon la revendication 1, caractérisés en ce que
A représente un atome d'hydrogène, un groupe alkyle en C₁-C₆, cycloalkyle en C₃-C₆, un groupe 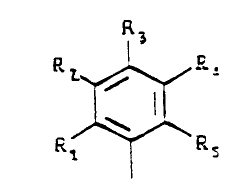 où
R₁ et R₅ représentent un atome d'hydrogène.

3. Dérivés de méthylènebishispidine selon la revendication 2, caractérisés en ce que R₈ représente un atome d'hydrogène, un groupe hydroxy, alcoxy en C₁-C₄, cyano, carboxy ou halogéno, R₂ et R₄ représentent, indépendamment l'un de l'autre et indépendamment de R₈, un atome d'hydrogène, un groupe hydroxy, alcoxy en C₁-C₄ ou alkyle en C₁-C₄.

4. Dérivés de méthylènebishispidine selon la revendication 1, caractérisés en ce que A représente un groupe 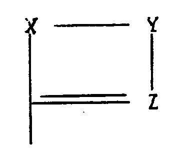 où
X représente un groupe -CH=CH-, NH, -CH=N-, un atome d'oxygène ou de soufre,
Y représente un groupe -CH=CH-, -CH=N- ou un atome de soufre, et
Z représente CH- ou un atome d'azote.

5. Dérivés de méthylènebishispidine selon l'une quelconque des revendications 1 à 4, caractérisés en ce que W représente un atome d'hydrogène, un groupe alkyle en C₁-C₄ ou carboxylester.

6. Médicament contenant au moins un dérivé de méthylènebishispidine selon l'une quelconque des revendications 1 à 5, et des matériaux de support et adjuvants usuels.

7. Utilisation de dérivés de méthylènebishispidine selon l'une quelconque des revendications 1 à 5 pour la préparation de médicaments destinés aux traitements préventif et curatif de lésions tardives diabétiques, de l'athérosclérose et de l'artériosclérose.

8. Procédé de préparation de dérivés de méthylènebishispidine de formule générale I, selon l'une quelconque des revendications 1 à 5, caractérisé en ce que l'on fait réagir l'hispidine (formule II) 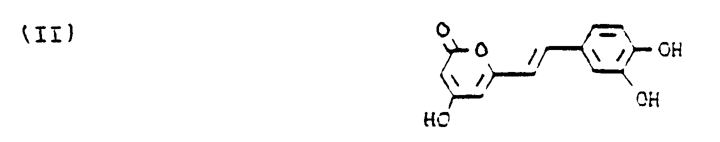 avec un aldéhyde ou une cétone de formule III 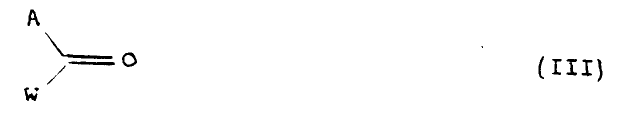 dans laquelle A et W présentent les significations mentionnées ci-dessus, par chauffage dans un solvant organique polaire, en présence de quantités catalytiques d'un acide minéral ou d'un solvant organique aprotique dipolaire, en présence de quantités catalytiques d'acétate de pipéridinium, avec déshydratation azéotropique.

9. Procédé selon la revendication 8, caractérisé en ce que l'on prépare l'hispidine (II) en faisant réagir le 3,4-dibenzyloxy-benzaldéhyde (IV) 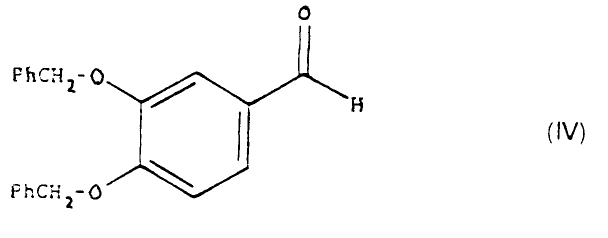 avec la 4-benzyloxy-6-méthyl-2H-pyran-2-one (V) 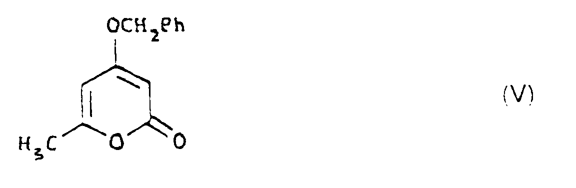 en présence de diisopropylamide de lithium, pour obtenir un composé aldol VI, 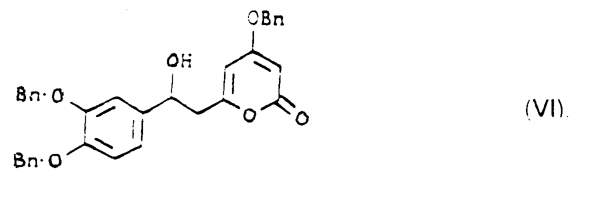 ensuite, on soumet celui-ci à une hydrogénation catalytique pour obtenir, par débenzylation, un composé tétrahydroxylé VII 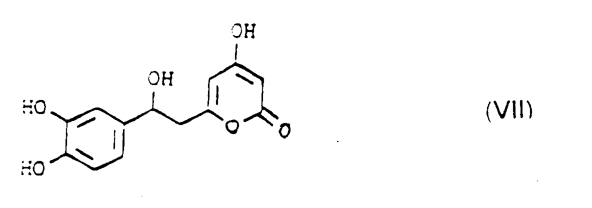 et enfin, on déshydrate le composé VII avec de l'acide minéral dilué en hispidine (II).
